# EUROPEAN PATENT SPECIFICATION

(11) **EP 0 980 688 B1**
(45) Date of publication and mention of the grant of the patent: **11.12.2002**
(21) Application number: 99104779.6
(22) Date of filing: 10.03.1999
(51) Int. Cl.: A61M 5/145

(54) **Automatic insulin syringe device with doctor mode**
Automatische Insulininjektionsspritze mit Arztmode
Seringue d'injection d'insuline automatique avec mode médécin

(30) Priority: 14.08.1998 KR 9833077
(43) Date of publication of application: 23.02.2000
(73) Proprietor: Choi, Soo Bong, Seoul (KR)
(72) Inventor: Choi, Soo Bong, Seoul (KR)
(74) Representative: Kuhnen & Wacker

(56) References cited:
- EP-A- 0 654 279
- WO-A-85/02546
- FR-A- 2 544 987
- US-A- 4 529 401
- US-A- 5 464 392
- US-A- 5 672 155

## Description

The present invention relates to an automatic insulin syringe device with a doctor mode.

### Description of the Prior Art

US-A-4 529 401 discloses a portable programmable insulin injector having the features of the preamble of claim 1 of the invention.

FR-A-2 544 987 discloses an insulin infusion device for the bedside of a patient. A special high rate of insulin can be selected with a button which is out of reach from the patients bed.

WO-A-85-02546 discloses a portable insulin injector where the basic programming of a memory for maximum doses is done from a detachable unit carried by the medical doctor.

Diabetes, which is a representative disease incidental to civilization, is known to affect more than one hundred million individuals worldwide at the present time, assuming there are about six billion people worldwide. For instance, in Korea, it is considered that diabetes affects about two million individuals. It is also estimated that 10% of patients with internal diseases suffer from diabetes in Korea. Diabetes is known as a disease not to be completely cured even though it can be controlled. If a diabetic patient fails to maintain a desired diabetic control, he may develop a complication, thereby endangering his life. For instance, in Korea, the death rate of diabetic patients is increasing gradually. In 1990, 11.5 individuals per population of one hundred thousand died. Thus, diabetes is known as a fatal disease.

Diabetes is a disease exhibiting a symptom of an increase in blood sugar level exceeding 140 mg/dl on an empty stomach or 200 mg/dl at a time 2 hours after a meal. The cause of such an increase in blood sugar level is not yet unclearly known. An abnormality in the production of insulin serving to conduct an assimilation of sugar is the only cause known heretofore. Such an abnormality may be an insulin deficiency caused by an insufficient quantity of insulin secreted by the β-cells of the pancreas. Otherwise, an increase in blood sugar level is caused by a degradation in the function of insulin occurring due to certain unknown reasons, thereby resulting in an insufficient assimilation of blood sugar, even though a desired quantity of insulin is normally secreted by the β-cells of the pancreas. Such a degradation in the function of insulin is called "insulin resistance". Diabetes resulting from an absolute insulin deficiency is called an "insulin-dependent diabetes" whereas diabetes resulting from an insufficient function of insulin in spite of a sufficient secretion of insulin is called a "non-insulin-dependent diabetes". Recently, an intermediate type between the insulin-dependent diabetes and non-insulin-dependent diabetes has also been discussed. It is difficult to accurately determine whether a diabetic patient is insulin-dependent or non-insulin-dependent. Known methods for treatment of diabetes are classified into dietary treatment, exercise treatment, medicinal therapy, and insulin injections. A pancreas transplant is also a conditional option.

Although the insulin injection method is a treatment used for patients with insulin-dependent diabetes, it may also be effective for patients with non-insulin-dependent diabetes. In accordance with the insulin injection method, it is normal that insulin is dispensed to a diabetic patient one or two times daily by injection. The quantity of insulin secreted in the human body is irregular in that an increased quantity of insulin is secreted three times daily before and after respective mealtimes while a reduced quantity of insulin is secreted at other times. For this reason, insulin is dispensed in an average quantity in accordance with the above mentioned insulin injection. This results in a high blood sugar level for a certain period of time after every mealtime because of an insulin deficiency while resulting in a low blood sugar level at night because of an excessive secretion of insulin. That is, the above mentioned insulin injection method involves an abnormal dispensation of insulin, thereby causing an abnormality of the body. The reason why conventional insulin injection methods cannot contribute to the prevention of any complications developed from diabetes is because they cannot control the dispensation of insulin in accordance with a variation in the quantity of insulin secreted in the body of a healthy person. To this end, improved treatment methods have been proposed. One method is to use an insulin pump (namely, a mechanical artificial pancreas) adapted to control the quantity of dispensed insulin using a computer in such a fashion that the dispensation of insulin approximates to the secretion of insulin in the body of a healthy person. Another method is a surgical operation method for transplanting the β-cells of the pancreas. In accordance with this pancreas transplant method, the β-cells of the pancreas of a healthy person are transplanted to a diabetic patient so that the diabetic patient normally secretes insulin to normally control his blood sugar level. However, the pancreas transplant method involves immunological rejection complications and other associated problems. This pancreas transplant method was studied in U.S.A. from 1974 and practically used by Professor Temberane at Yaeil University in U.S.A from 1979.

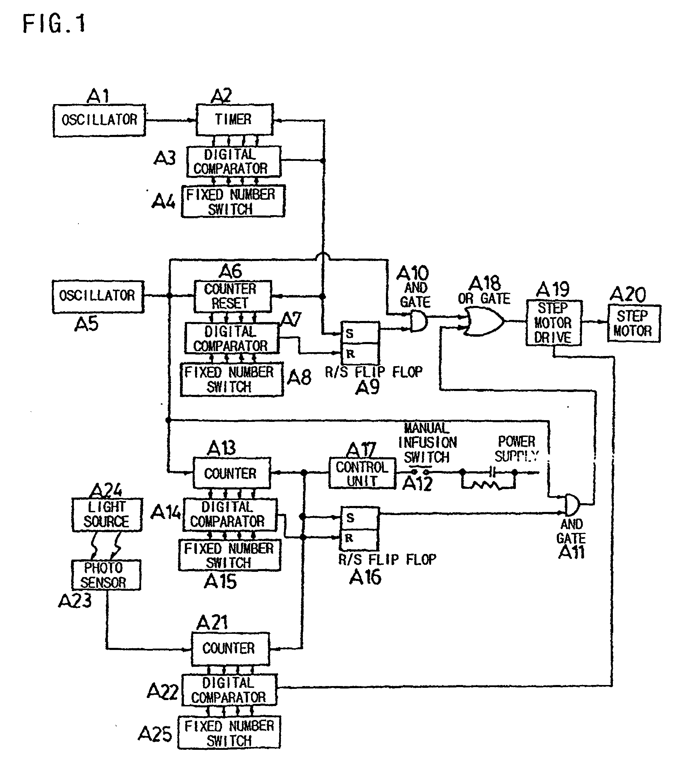

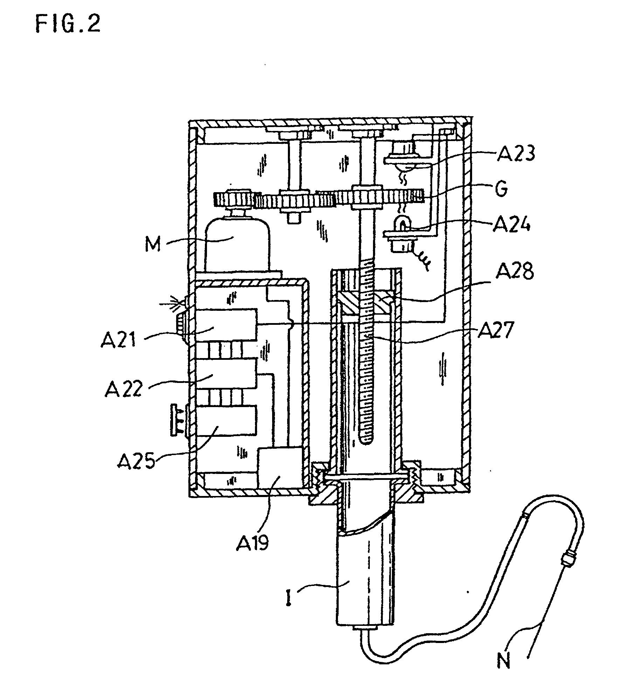

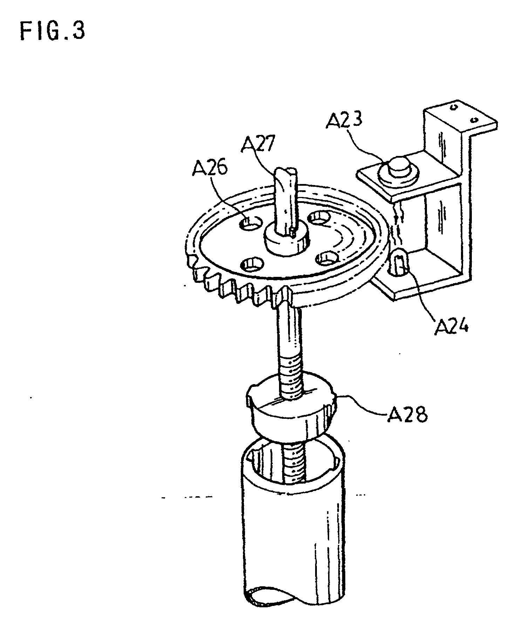

Automatic syringe devices, which enable an injection of liquid medicine for a prolonged time, are well known. Such automatic syringe devices are called "insulin pumps", "insulin syringe devices", or "automatic insulin syringe devices". Typically, known automatic syringe devices have a configuration in which a push means for pushing a syringe piston is coupled to a housing receiving an injection syringe. For example, such automatic syringe devices are disclosed in Japanese Utility Model Laid-open Publication No. Sho. 52-3292 and U.S. Patent No. 4,417,889. The syringe device disclosed in Japanese Utility Model Laid-open Publication No. Sho. 52-3292 has inconvenience in carrying it because it has an injector mounted outside a basic case, thereby requiring a double case structure. In order to solve such a disadvantage, an automatic syringe device requiring no double case structure has been proposed, as in the above mentioned U.S. Patent No. 4,417,889. Fig. 1 illustrates a control circuit of the automatic syringe device disclosed in U.S. Patent No. 4,417,889, respectively. Referring to Fig. 1, the output of an oscillator A1 is coupled to a timer A2 which is, in turn, coupled at its output to a digital comparator A3. The digital comparator A3 also receives an output from a fixed number switch A4. The output of the digital comparator A3 is connected to a counter A6 and an R/S flip-flop A9. Another oscillator A5 is also provided which has an output coupled to counters A6 and A13, and AND gates A10 and A11. The flip-flop A9 is reset by an output from a digital comparator A7. Another R/S flip-flop A16 is also provided which is reset by an output from a digital comparator A14 coupled to the counter A13. A control unit A17 is also coupled to the counter A13. The control unit A17 serves to activate the counter A13 in accordance with an operation of a manual infusion switch A12. The control unit A17 applies its output to the counters A13 and A16. The output from the control unit A17 is also sent to a counter A21. The output of the counter A21 is coupled to a digital comparator A22 which is, in turn, coupled to a step motor driver A19 for driving a step motor A20. The output of the flip-flop A16 is coupled to one input of the AND gate A11, which is also coupled at the other input thereof to the oscillator A5. The output of the AND gate A11 is coupled to one input of an OR gate A18. Fixed number switches A15 and A25 are connected to the digital comparators A14 and A22, respectively. Each of the fixed number switches A4, A8, A15, and A25 has five protruding insert bars and serves to provide a reference value for an associated one of the digital comparators A3, A7, A14, and A22. A light source A24 and a photo sensor A23 are coupled to the counter A21 in order to provide sensing results thereof to the counter A21, respectively. Referring to Figs. 2 and 3, the arrangements of the light source A24 and photo sensor A23 are illustrated. As shown in Figs. 2 and 3, the light source A24 and photo sensor A23 are arranged in such a fashion that they face each other while being vertically spaced from each other. A gear plate, which is included in a gear mechanism G, is interposed between the light source A24 and photo sensor A23. The gear plate has a plurality of through holes A26 uniformly spaced from one another in a circumferential direction, as shown in Fig. 3. The gear plate is fixedly fitted around a gear shaft A27 having a screw portion. A piston plate A28 is threadedly coupled to the gear shaft A27 in the form of a nut in such a fashion that it slides along the screw portion of the gear shaft A27 when the gear shaft A27 rotates. The rotation of the gear shaft A is carried out by a drive force from the motor A20 transmitted via the gear mechanism G. The driving of the motor A20 is controlled by the operations of the counter A21, digital comparator A22, switch A25, and motor drive A19. All the above mentioned elements of the syringe device are received in a housing. In particular, the light source A24 and photo sensor A23 are fixedly mounted at an upper portion of the housing by means of a bracket fixed to the housing. In this syringe device, a liquid medicine, such as insulin, contained in a syringe I is outwardly injected through an injection needle N connected to the syringe I, by a slide movement of the piston plate A28. In such a syringe device, however, the housing and syringe I thereof are exposed to ambient air. As a result, moisture and water are likely to penetrate into the syringe device. For this reason, there is inconvenience in that if the user desires to take a shower while the syringe is in place, then the housing should be contained in a separate sealing case.

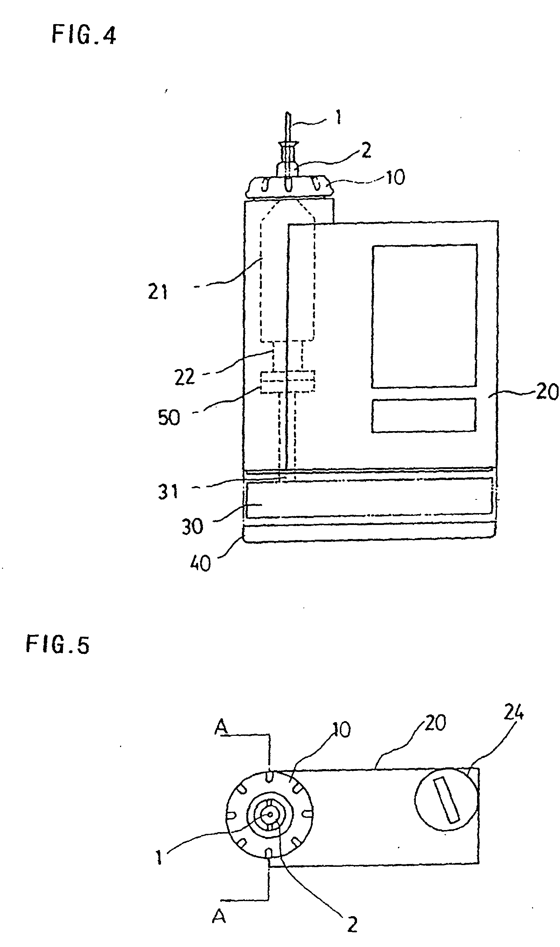

In order to solve such a problem, a sealable syringe device has been proposed by the applicant. Such a sealable syringe device is illustrated in Fig. 4 which is a front view. Referring to Fig. 4, the syringe device includes a cover 10 sealably coupled to the upper end of a housing 10, and a bottom cover 40 sealably coupled to the lower end of the housing 10. A connector 2, to which a feeding tube is integrally connected, is threadedly coupled to the cover 10. The connector 2 communicates with a syringe 21 received in the housing 10. A piston 22 is slidably fitted in the syringe 21. A liquid medicine to be syringed is contained in the syringe 21. A power transmission means 30 is mounted on the bottom surface of the housing 20. The power transmission means 30 has a rotating shaft 31 to which a disc type push means 50 is threadedly coupled. The disc type push means 50 moves vertically by a rotation of the rotating shaft 31, thereby vertically moving the piston 22.

Referring to Fig. 5, which is a plan view of Fig. 4, the cover 10, to which the connector 2 connected with the feeding tube 1 is connected, is arranged on the left portion of the upper surface of the housing 20. A battery cover 24 is arranged on the right portion of the upper surface of the housing 20.

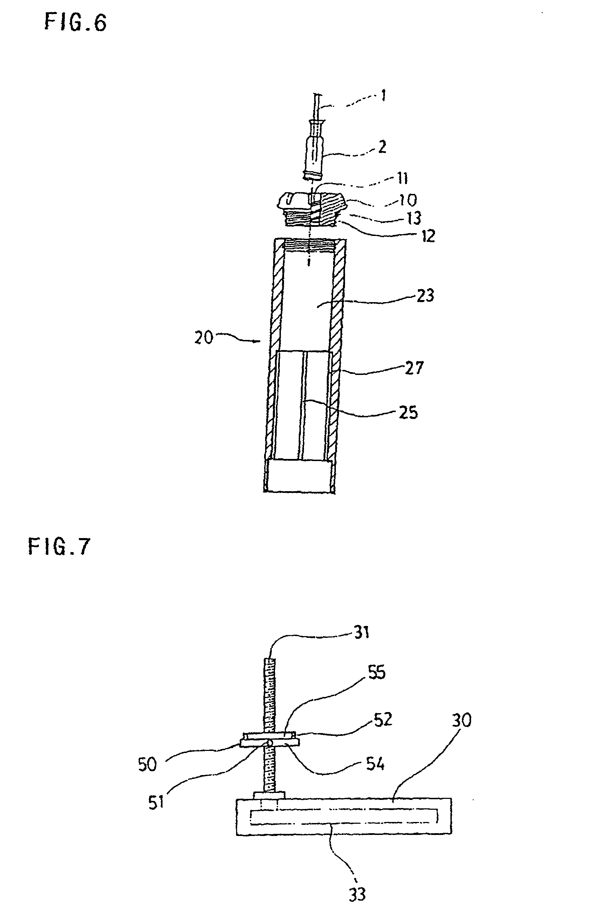

Fig. 6 is a cross-sectional view taken along the line A - A of Fig. 5. As shown in Fig. 6, the cover 10 is centrally provided with a threaded hole 11 in which the connector 2 is threadedly fitted at its lower end. The cover 10 is also provided at its lower end with a bolt portion 12 threadedly fitted in the upper end of the housing 20. A packing 13 is fitted around the bolt portion 12 of the cover 10 between the lower end of the cover 10 and the upper end of the housing 20. A syringe receiving chamber 23 is defined in the interior of the housing 20. The push means 50 is fitted in the lower end of the housing 20 in such a fashion that it slides vertically in the housing 20. The housing 20 is also formed at its inner surface with a vertical push means guide groove 25 adapted to guide a vertical movement of the push means 50 and vertical piston guide grooves 27 adapted to guide a vertical movement of the piston 22.

Fig. 7 shows a detailed configuration of the push means 50 threadedly coupled to the rotating shaft 31 of the power transmission means 30, along with a detailed configuration of the power transmission means 30. As shown in Fig. 7, the push means 50 includes a lower disc 54 threadedly coupled to the rotating shaft 31 in such a fashion that it slides vertically along the rotating shaft 31. The lower disc 54 is provided at its periphery with a guide protrusion 51 engaged in the guide groove 25 of the housing 20 and adapted to guide the vertical movement of the lower disc 54. The push means 50 also includes an upper disc 55 integrally formed with the lower disc 54. The upper disc 55 is provided at its periphery with an engagement means 52. The upper disc 55 is fitted in a sleeve plate 26 fixed to the lower end of the piston 22 in such a manner that its engagement means 52 engages with a mating engagement means formed on the inner peripheral surface of the sleeve plate 26. The sleeve plate 26 is also provided at its outer peripheral surface with protrusions engaging with the guide grooves 27 respectively. The power transmission means 30 includes a reduction mechanism 33 for transmitting the rotating force of a motor (not shown) to the rotating shaft 31 in a speed-reduced manner.

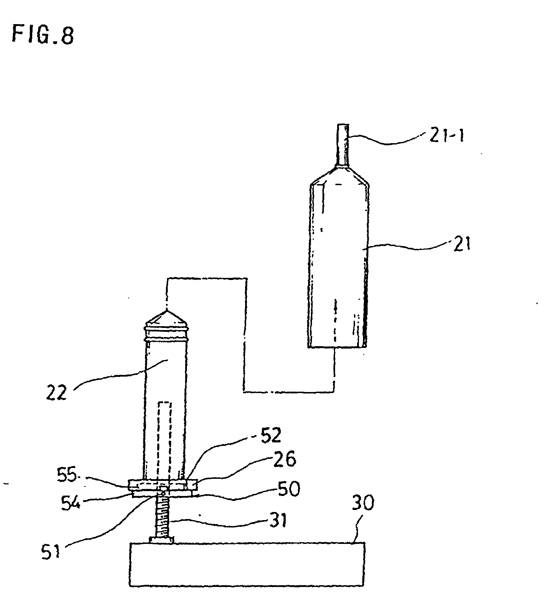

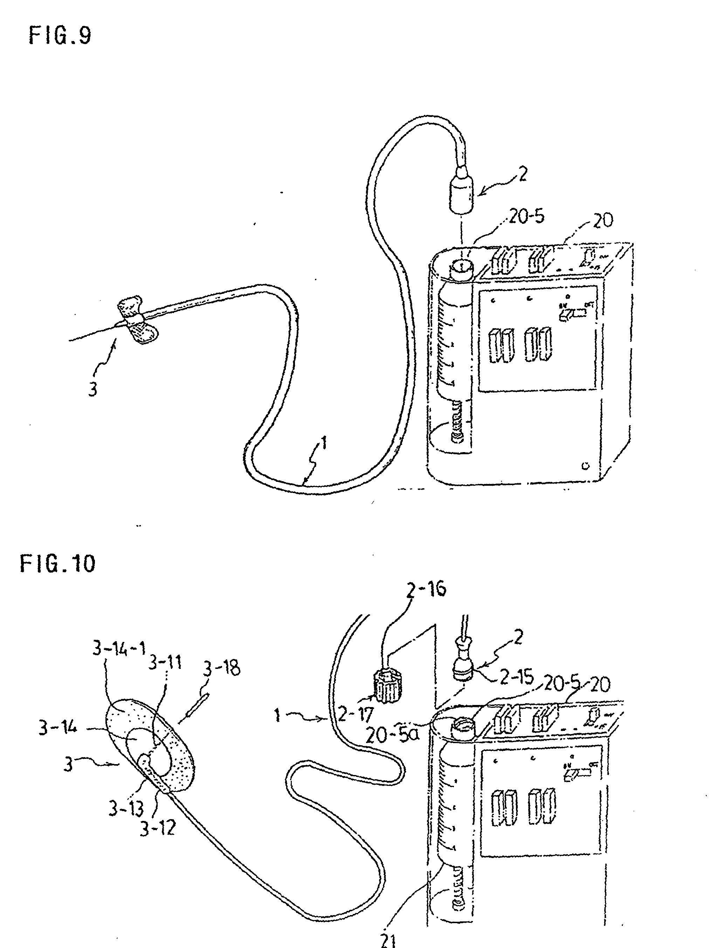

In order to use the syringe device having the above mentioned configuration, the piston 22, which is in a state separated from the housing 20, is first fitted in the syringe 21 which is also in a state separated from the housing 20, in such a manner that it is completely inserted into the syringe 21, as shown in Figs. 8 and 10. In this state, a disposable injection needle (not shown) is fitted onto the tip 21-1 of the syringe 21. Thereafter, the injection needle is penetrated into the interior of a phial through the plug of the phial. In this state, the piston 22 is pulled to suck a liquid medicine (for example, insulin) contained in the phial into the syringe 21.

The piston 22, which is in a state fitted in the syringe 21 containing the liquid medicine, is then inserted into the syringe receiving chamber 23 of the housing 20 in such a manner that it is seated on the push means 50. Subsequently, the cover 10 is threadedly coupled to the upper end of the housing 23. The connector 2 is then threadedly fastened to the cover 10. As the connector 2 is threadedly fastened to the cover 10, it is fitted onto the syringe tip 21-1. Thus, the syringe 21 is maintained in a sealed state in the housing 20. When the motor (not shown) drives under the above condition, the push means 50 moves upwardly, thereby upwardly pushing the piston 22. As a result, the liquid medicine contained in the syringe 21 is outwardly injected from the syringe 21. At this time, the upward movement of the push means 50 is accurately carried out because its guide protrusion 51 engages with the guide groove 25. Since the engagement means 52 of the push means 50 engages with the mating engagement means of the sleeve plate 26 integrally formed with the lower end of the piston 22, the upward movement of the piston 22 is also accurately carried out.

In such a syringe device, it is necessary to set the initial height or vertical position of the lower disc 54 of the push means 50 every time the syringe 21 filled with a liquid medicine is inserted into the housing 20, in order to allow the piston 22 to be accurately seated on the upper disc 55 of the push means 50. However, it is difficult to accurately set a desired initial vertical position of the lower disc 54.

Meanwhile, Fig. 9 illustrates an example of a conventional injection needle unit used for portable automatic syringe devices enabling a prolonged injection of a liquid medicine. As shown in Fig. 9, the injection needle unit includes a feeding tube 1, a "-" shaped straight injection needle member (called a "straight butterfly-shaped injection needle") 3 connected to one end of the feeding tube 1, and a connector 2 connected to the other end of the feeding tube 1.

In order to use such an injection needle unit, the user himself slantly penetrates the injection needle member 3 into the subcutaneous tissue while observing the penetration of the injection needle member 3 with the naked eye. The reason why the user observes the penetration of the injection needle member 3 with the naked eye is because the injection needle member 3 has a straight shape. However, such an observation is very uncomfortable.

Furthermore, when the straight butterfly-shaped injection needle member 3 penetrates the subcutaneous tissue of the user on a slanted angle, its tip may be easily blocked by the subcutaneous tissue because the subcutaneous tissue is a multilayer tissue. As a result, the above mentioned conventional injection needle unit has a drawback in that it is difficult to smoothly inject the liquid medicine, namely, insulin.

The straight butterfly-shaped injection needle member 3 is also likely to move in the subcutaneous tissue of the user because it penetrates the subcutaneous tissue of the user on a slanted angle. In this case, the subcutaneous tissue may be damaged. In severe cases, blood may flow out of the subcutaneous tissue. The user may also feel a severe pain.

As mentioned above, the conventional injection needle unit has a drawback in that it is difficult to smoothly inject insulin because the injection needle member 3, which penetrates the subcutaneous tissue of the user on a slanted angle, may be easily blocked at its tip by the subcutaneous tissue. To this end, the feeding tube of such a conventional injection needle unit inevitably has an increased diameter. However, such a feeding tube having an increased diameter results in a possibility of an excessive insulin injection. In addition, this may result in wastage of expensive insulin. For instance, where it is desired to inject insulin into the user using an automatic syringe device equipped with the above mentioned injection needle unit, it is necessary to completely vent air existing in the feeding tube 1 and injection needle member 3 before penetrating the injection needle member 3 into the subcutaneous tissue of the user. To this end, insulin, which is contained in the syringe device, is outwardly discharged through the feeding tube 1 and injection needle member 3, thereby venting air. In this case, a large amount of insulin is wasted where the conventional injection needle unit having the diameter-increased feeding tube is used.

The use of such a diameter-increased feeding tube also results in an increase in the manufacturing costs.

In the case of the injection needle unit illustrated in Fig. 9, its connector 2 is simply fitted onto a connector portion 20-5 of the syringe device housing 20. For this reason, the connector 2 may be incidentally separated from the connector portion 20-5 of the housing 20.

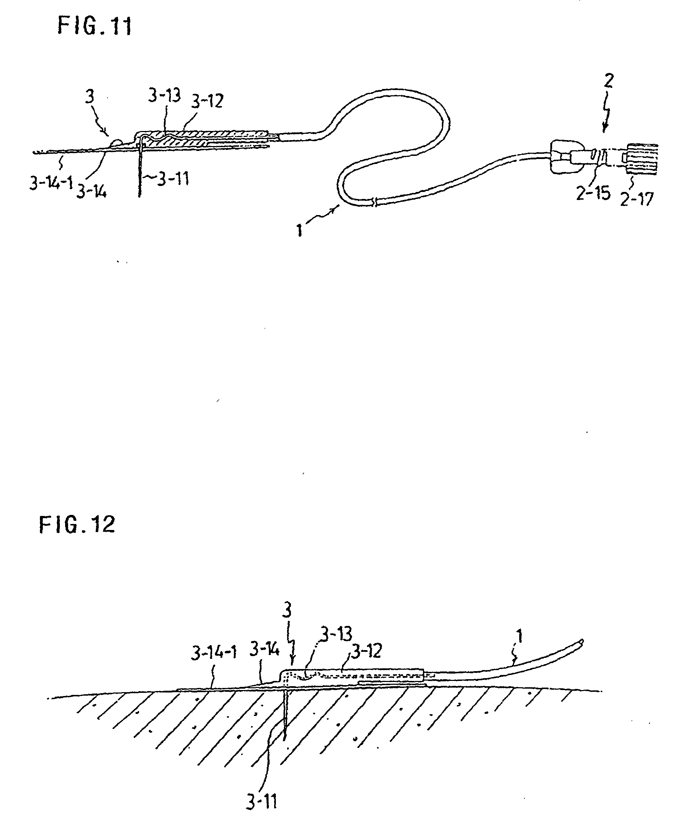

In order to solve this problem, an injection needle unit has been proposed which has a configuration capable of preventing a separation of its connector. Such an injection needle unit is illustrated in Figs. 10 and 11, respectively.

As shown in Figs. 10 and 11, the injection needle. unit includes a feeding tube 1, an injection needle member 3 connected to one end of the feeding tube 1, and a connector 2 connected to the other end of the feeding tube 1.

In the case of the injection needle unit shown in Figs. 10 and 11, the injection needle member 3 has an "L" shaped injection needle 3-11. This injection needle 3-11 has a first portion, namely, a horizontal portion, fitted in a connecting rib 3-12 integrally formed with one end of the feeding tube 1, and a second portion, namely, a vertical portion, provided with a needle tip. The injection needle 3-11 is provided with a curved portion 3-13 at its horizontal portion fitted in the connecting rib 3-12, as shown in Fig. 11. A depressing member 3-14 is integrally formed with the connecting rib 3-12 in such a fashion that the injection needle 3-11 protrudes perpendicularly from the depressing member 3-14. The depressing member 3-14 is depressed against the skin of the user upon penetrating the injection needle member 3 into the subcutaneous tissue. A bacterial infection prevention member 3-14-1, which is made of a disinfected nonwoven fabric, is attached to the surface of the depressing member 3-14 which comes into contact with the skin of the user upon penetrating the injection needle unit 3 into the subcutaneous tissue. The feeding tube 1 of Figs. 10 and 11 has a reduced diameter and an increased length, as compared to that of Fig. 9. The connector 2, which is connected to the other end of the feeding tube 1, has a male thread 2-15. The connector 2 is protected by a protection cap 2-17 which has a female thread 2-16 threadedly coupled to the male thread 2-15 of the connector 2. In use, the connector 2 is threadedly coupled to a connector portion 20-5 of a housing 20 included in an automatic insulin syringe device. The connector portion 20-5 of the housing 20 has a female thread 20-5a threadedly coupled to the male thread 2-15 of the connector 2. In Fig. 10, the reference numeral "3-18" denotes a needle protection cap, and the reference numeral "21" is a syringe.

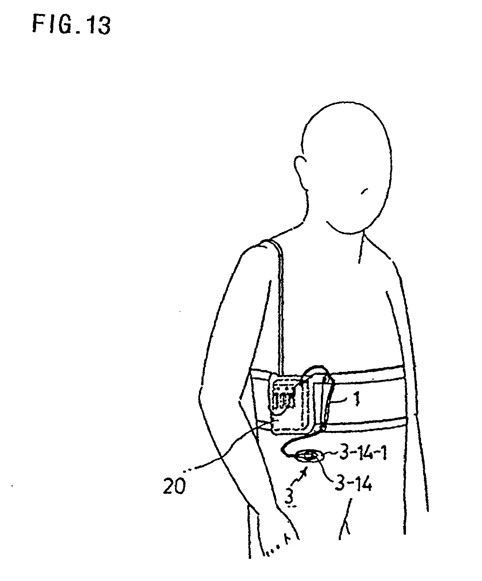

Where it is desired to inject insulin contained in the automatic insulin syringe device using the above mentioned injection needle unit, the protection cap 2-17 is first separated from the connector 2, which is, in turn, threadedly coupled to the connector portion 20-5 of the housing 20. Thereafter, the needle protection cap 3-18 is separated from the injection needle 3-11. The user then penetrates the injection needle 3-11 into the subcutaneous tissue while depressing the depressing member 3-14 against the skin by fingers. At this time, the injection needle 3-11 penetrates vertically into the subcutaneous tissue of the user because it has an "L" shape. Accordingly, the user can carry out the penetration of the injection needle 3-11 instantaneously without any observation with the naked eye. Therefore, the user feels little pain upon penetrating the injection needle 3-11 into the subcutaneous tissue. By virtue of such a configuration of the injection needle unit 3, the automatic insulin syringe device can be conveniently used, as shown in Fig. 13. Since the injection needle 3-11 penetrates vertically into the subcutaneous tissue of the user by virtue of its "L" shape, there is no phenomenon that the injection needle 3-11 is blocked at its tip by the subcutaneous tissue of the user. Thus, the injection of insulin is smoothly carried out. Accordingly, the feeding tube can have a reduced diameter and an increased length. Since the feeding tube 1 has a reduced diameter, it is possible to minimize the wastage of insulin occurring upon venting air existing in the feeding tube 1 and injection needle 3-11 and to reduce the manufacturing costs. Since the feeding tube 1 also has an increased length, it is possible to extend the range of the position of the injection needle 3-11 on the body of the user. Accordingly, it is possible to achieve convenience in use. Since the bacterial infection prevention member 3-14-1, which is made of a disinfected nonwoven fabric, is attached to the depressing member 3-14, it is possible to prevent the depressing member 3-14 from coming into direct contact with the skin of the user upon penetrating the injection needle unit 3 into the subcutaneous tissue. Accordingly, it is possible to prevent the user from being infected. Since the injection needle 3-11 penetrates vertically into the subcutaneous tissue of the user by virtue of its "L" shape, as mentioned above, it hardly moves in the subcutaneous tissue even when an external force is applied thereto. Accordingly, there is no damage of the subcutaneous tissue. Of course, there is no phenomenon that the blood flows out of the subcutaneous tissue. The user also does not feel any pain.

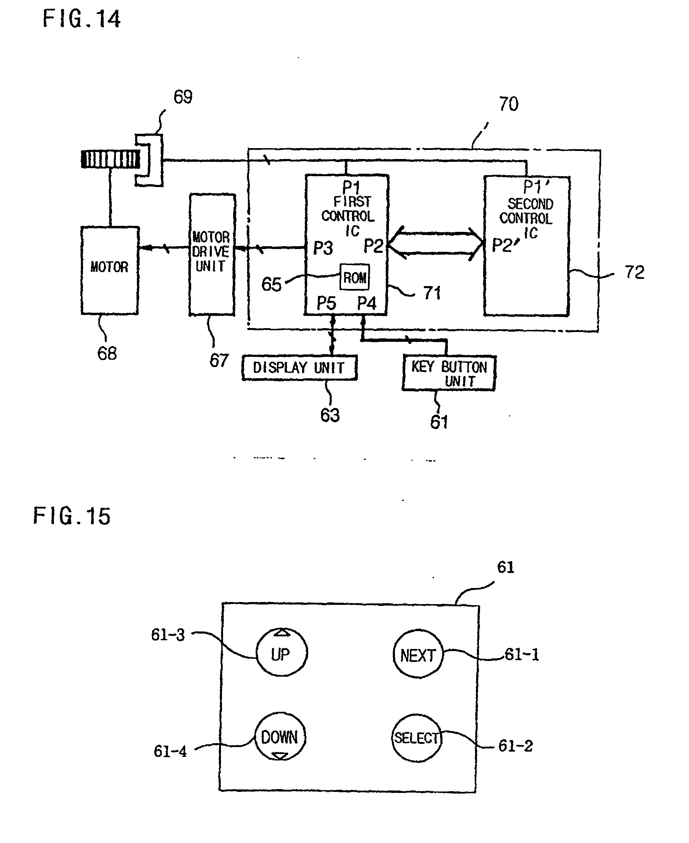

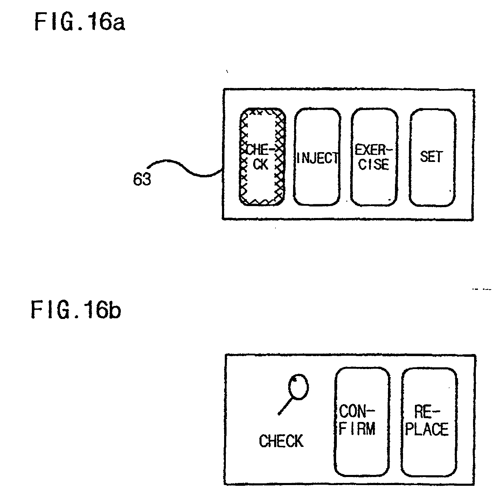

Referring to Fig. 14, a control circuit for the above mentioned syringe device is illustrated. As shown in Fig. 14, the control circuit includes a key button unit 61 for generating a key signal adapted to select a desired control function, a control unit 70 provided with functions of a microcomputer and adapted to carry out a control operation in response to the key signal generated from the key pad unit 61, a display unit 63 adapted to display data outputted from the control unit 70, a ROM 65 adapted to store a variety of data and programs, a motor drive unit 67 adapted to drive a motor 68 under the control of the control unit 70, and a photocoupler 69 adapted to sense a rotation of the motor 68. The rotation of the motor 68 is controlled by the motor drive unit 67. Preferably, the control unit 70 comprises a first control IC 71 and a second control IC 72 having the same function. The first and second control ICs 71 and 72 monitor each other so that when one of them operates abnormally, the other control IC serves to stop the overall operation of the syringe device. Thus, the control unit 70 allows the syringe device to operate only in a stable state. For each of the control ICs 71 and 72, a one-chip microcomputer may be used. In Fig. 14, the reference characters P2 and P2' denote respective ports of the first and second control ICs 71 and 72 coupled to each other via data and bus lines. An example of the key button unit 61 is illustrated 15 whereas examples of the display unit 63 are illustrated in Figs. 16a and 16b, respectively. For the motor 68, a stepping motor or servo motor may be used.

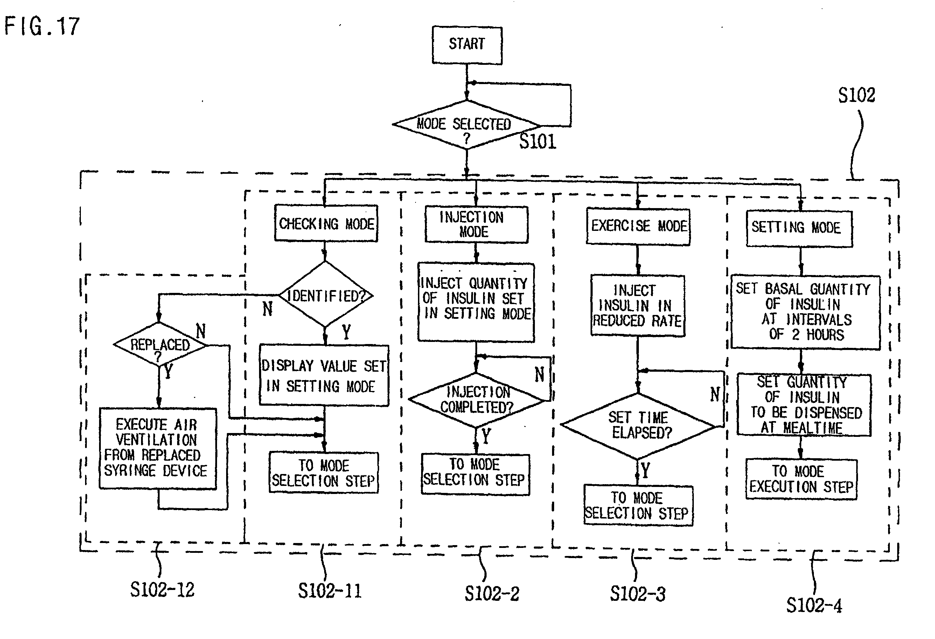

Referring to Fig. 17, an algorithm for controlling the syringe device using the above mentioned control circuit is illustrated. This algorithm is stored in the ROM 65 of the control circuit. Now, a conventional method for controlling the syringe device will be described in conjunction with Fig. 17.

When a power switch (not shown in the block diagram of Fig. 14) is switched on, a mode selection window including a plurality of window tabs respectively indicative of a variety of operation modes is displayed on the screen of the display unit 63. In this state, the user repeatedly manipulates a "NEXT" switch 61-1 shown in Fig. 15 in such a fashion that a cursor, which is distinguished from the window tabs by a chrominance, color or symbol size different from that of the window tabs, is positioned at a selected one of the window tabs. When the cursor is positioned at a selected window tab, the user manipulates a select switch 61-2 to be switched on. At a first step S101, accordingly, it is determined whether or not a select switch 61-2 shown in Fig. 15 is switched on. When a desired operation mode is selected by a manipulation of the select switch 61-2 by the user, a second step S102 is executed to perform an operation corresponding to the selected operation mode. Where a checking mode is selected at second step S102, that is, when the select switch 61-2 is switched on in a state in which the cursor is positioned at a window tab indicative of the checking mode shown in Fig. 16a, the procedure proceeds to a routine S102-11. In the routine S102-11, it is first determined whether or not a confirmation command for confirming the amount of insulin to be dispensed per hour, namely, the insulin injection rate, which has been set in a setting mode, is generated. Where it is determined that the confirmation command is generated, that is, where the select switch 61-2 is switched on in a state in which the cursor is positioned at a "CONFIRM" window tab displayed upon selecting the checking mode, as shown in Fig. 16b, the insulin injection rate set in the setting mode is displayed in the form of a graph so that it is identified by the user. Thereafter, the procedure returns to first step S101. This can be achieved by depressing again the select switch 61-2 even though various methods may be used. Where it is determined that no confirmation command is generated in the routine S102-11, the procedure proceeds to a routine S102-12. In the routine S102-12, it is first determined whether or not a replacement command is generated, that is, whether or not the select-switch 61-2 is switched on in a state in which the cursor is positioned at a window tab indicative of the replacement mode. When the replacement command is generated, an air ventilation for venting air existing in the syringe device is carried out. Thereafter, the procedure returns to first step S101. When no replacement command is generated, the procedure returns directly to first step S101.

Where an injection mode is selected at second step S102, that is, when the select switch 61-2 is switched on in a state in which the cursor is positioned at a window tab indicative of the injection mode shown in Fig. 16a by repeatedly depressing the "NEXT" switch 61-1, the procedure proceeds to a routine S102-2. In the routine S102-2, a well-known insulin injection procedure is executed. After the completion of the insulin injection, a signal is generated from a signal means (not shown) to inform the user of the completion of the insulin injection while stopping the injection operation. Thereafter, the procedure returns to step S101.

When an exercise mode is selected at second step S102, that is, when the select switch 61-2 is switched on in a state in which the cursor is positioned at a window tab indicative of the exercise mode shown in Fig. 16a by repeatedly depressing the "NEXT" switch 61-1, the procedure proceeds to a routine S102-3. In the routine S102-3, an insulin injection is executed in a rate reduced from the set injection rate for a predetermined period of time (for example, one hour). After the predetermined period of time elapses, the procedure returns to first step S101. The reduced insulin injection rate should be determined depending on the body constitution of the user. In the exercise mode, accordingly, the reduced insulin injection rate is appropriately determined by manipulating "UP and "DOWN" switches 61-3 and 61-4 shown in Fig. 15. The reduced insulin injection rate may be displayed in the form of a percentage of the set insulin injection rate.

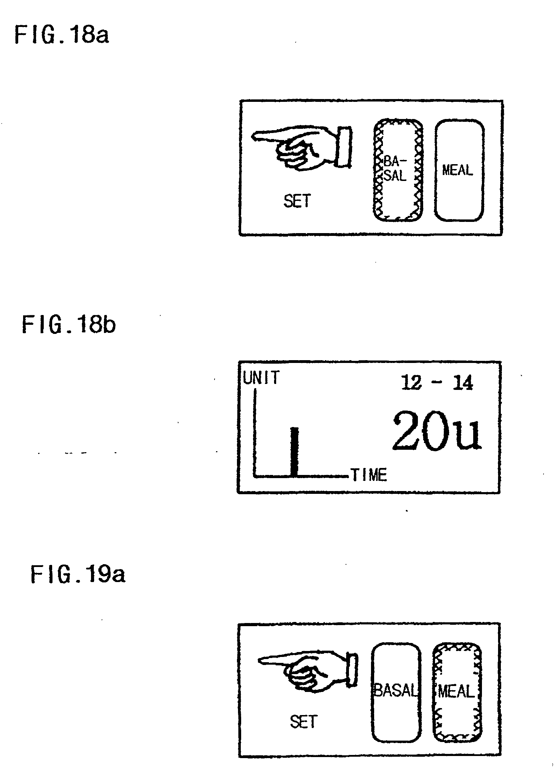

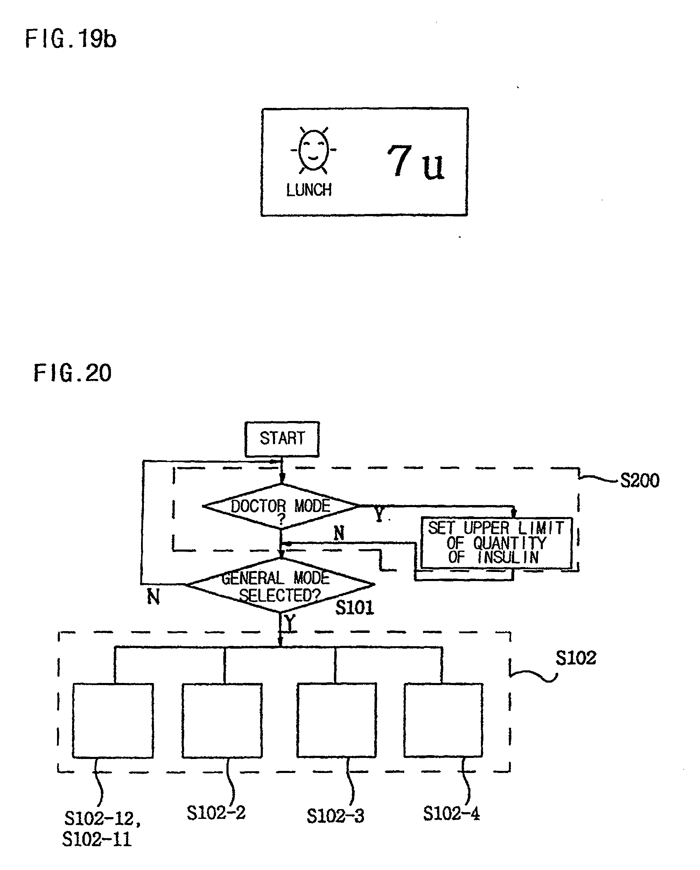

On the other hand, where a setting mode is selected at second step S102, that is, when the select switch 61-2 is switched on in a state in which the cursor is positioned at a window tab indicative of the setting mode shown in Fig. 16a by repeatedly depressing the "NEXT" switch 61-1, the procedure proceeds to a routine S102-4. In the routine S102-4, a window shown in Fig. 18a is displayed. Where the cursor is positioned at a window tab "BASAL" indicative of a basal control for the insulin injection rate, the select switch 61-2 shown in Fig. 15 is depressed, thereby setting a basal insulin injection rate. For example, the setting of the basal insulin injection rate may be achieved by manipulating the "UP" and "DOWN" switches 61-3 and 61-4 shown in Fig. 15, thereby setting a desired basal insulin injection by a value expressed in terms of units, as shown in Fig. 18b. In this case, a minimum insulin injection rate corresponds to one unit (1u). The setting of the basal insulin injection rate may be carried out at intervals of 2 hours. Basal data of such a display pattern output is previously stored in the ROM 65 as a memory means. When the control unit 70 generates a desired data address signal, the ROM 65 outputs to the display unit 63 data stored in a memory location corresponding to the generated data address signal. Since such a technique is well known, no further description will be made. On the other hand, when a window tab "MEAL" of Fig. 19b indicative of a control for the insulin injection rate depending on the quantity of meals is selected, windows tabs respectively indicative of a mealtime and an insulin injection rate are displayed, as shown in Fig. 19c. In Fig. 19c, a lunchtime indicator and an associated insulin injection rate are displayed. Using the window tabs, a desired mealtime and a desired insulin injection rate according to the body configuration of the user are then selected. Thereafter, the procedure returns to second step S102.

The automatic syringe device having the above mentioned function has an advantage in that the user can adjust the quantity of insulin to be injected in accordance with his body configuration. However, where a person not diligently maintaining the necessary function of this device or an unskilled person uses such an automatic syringe device, he may erroneously adjust the quantity of insulin to be injected. Where the quantity of insulin to be injected is set to an excessive level that is fatal to the user, it may result in harmful effects (for example, a low blood sugar level) injurious to health.

### SUMMARY OF THE INVENTION

Therefore, an object of the invention is to provide a method for controlling the operation of an automatic syringe device, which is capable of allowing only an authorized doctor to set an upper limit of the quantity of liquid medicine to be dispensed to a patient while allowing the patient to adjust the quantity of liquid medicine to be dispensed to him in a range not beyond the set upper limit of the liquid medicine quantity, thereby eliminating harmful effects resulting from an excessive injection of the liquid medicine.

In order to accomplish the above mentioned object, the present invention provides an automatic syringe device, in which a control unit equipped in the syringe device recognizes selection of a doctor mode when a key button unit equipped in the syringe device is manipulated in a manner different from those in the selection of other operation modes, and carries out a control for preferentially selecting the quantity of liquid medicine set in the doctor mode over that set in other operation modes.

Controlling the operation of an automatic insulin syringe device, which selects one from operation modes of the automatic insulin syringe device and executes the selected operation mode, may consist of the following steps: preliminary step of allowing access to a doctor mode by an authorized doctor, who uses a secret code enabling access to said doctor mode and is familiar with a control method associated with said doctor mode, and allowing the authorized doctor to set an upper limit of the quantity of insulin to be dispensed to a patient using said syringe device; mode selection step of determining whether or not an operation mode of said syringe device other than said doctor mode is selected after said upper limit has been set in said preliminary step or when no doctor mode is selected; and mode execution step of executing said operation mode selected at said mode selection step while allowing an adjustment of the quantity of insulin to be dispensed under the condition in which said upper limit is preferentially selected as a setting value in said adjustment.

### BRIEF DESCRIPTION OF THE DRAWINGS

Other objects and aspects of the invention will become apparent from the following description of embodiments with reference to the accompanying drawings in which:
Fig. 1 is a block diagram illustrating a control circuit used in a conventional automatic syringe device;
Fig. 2 is a cross-sectional view illustrating a structure of the automatic syringe device;
Fig. 3 is a perspective view illustrating the installation of a photo sensor in the automatic syringe device;
Fig. 4 is a front view illustrating another conventional automatic syringe device;
Fig. 5 is a plan view of Fig. 4;
Fig. 6 is a cross-sectional view taken along the line A - A of Fig. 5;
Fig. 7 is a view illustrating a conventional power transmission means incorporated in the syringe device of Fig. 6;
Fig. 8 is an exploded view illustrating a conventional push means incorporated in the syringe device of Fig. 7;
Fig. 9 is a perspective view illustrating an example of a conventional injection needle unit used for portable automatic syringe devices;
Fig. 10 is a perspective view illustrating another conventional injection needle unit;
Fig. 11 is a partially-broken plan view illustrating the injection needle unit of Fig. 10;
Fig. 12 is an enlarged view illustrating a using condition of the injection needle unit of Fig. 10;
Fig. 13 is a perspective view illustrating a using condition of the injection needle unit of Fig. 10;
Fig. 14 is a block diagram illustrating a control circuit for an automatic syringe device to which the present invention is applied;
Fig. 15 is a view illustrating a key button unit of the control circuit to which the present invention is applied;
Fig. 16a is a view illustrating an initial mode selection window displayed on the display screen of a display unit of the control circuit in accordance with the present invention;
Fig. 16b is a view illustrating a window displayed on the display screen when a window tab of Fig. 16a corresponding to a checking mode is selected;
Fig. 17 is a flow chart illustrating a conventional method for controlling the operation of an automatic syringe device in association with the control circuit of Fig. 14;
Fig. 18a is a view illustrating a window displayed on the display screen and used to set a basal quantity of insulin to be dispensed;
Fig. 18b is a view illustrating a window displayed on the display screen when a setting of the basal insulin quantity is selected from the window of Fig. 18a, the window displaying the quantity of insulin to be dispensed;
Fig. 19a is a view illustrating a window displayed on the display screen wand used to set a quantity of insulin to be dispensed at a mealtime;
Fig. 19b is a view illustrating a window displayed on the display screen when a setting of the insulin quantity to be dispensed at the mealtime is selected from the window of Fig. 19a, the window displaying the quantity of insulin to be dispensed at the mealtime;
Fig. 20 is a flow chart illustrating a method for controlling the operation of an automatic syringe device in association with the control circuit of Fig. 14 in accordance with the present invention;
Fig. 21a is a view illustrating a window displayed on the display screen for an execution of a preliminary function, when a doctor mode is selected, in accordance with the present invention;
Fig. 21b is a view illustrating a window displayed on the display screen when access to the doctor mode is achieved, the window including a "TIME" window tab and an "UPPER LIMIT" window tab;
Fig. 22a is a view illustrating the window of Fig. 21b in which the "TIME" window tab is selected by shifting a cursor in the window of Fig. 21b;
Fig. 22b is a view illustrating a time adjustment window displayed on the display screen when the "TIME" window tab is selected, the time adjustment window displaying a clock along with a setting time;
Fig. 23a is a view illustrating the window of Fig. 21b in which the "UPPER LIMIT" window tab is selected; and
Fig. 23b is a view illustrating an upper limit adjustment window displayed on the display screen when the "UPPER LIMIT" window tab is selected as shown in Fig. 23a.

### DESCRIPTION OF THE PREFERRED EMBODIMENTS

Fig. 20 is a flow chart illustrating a method for controlling the operation of an automatic syringe device, for example, an insulin pump, in accordance with the present invention. As shown in Fig. 20, the control method of the present invention includes a preliminary step S200 of allowing access to a doctor mode by an authorized doctor, who uses a secret code enabling access to said doctor mode and is familiar with a control method associated with said doctor mode, and allowing the authorized doctor to set an upper limit of the quantity of insulin to be dispensed to a patient using the insulin pump, and a mode selection step S101 of determining whether or not a general mode is selected after the upper limit has been set in the preliminary step S200 or when no doctor mode is selected, and a mode execution step S102 of executing an operation mode selected at the mode selection step S101 while allowing an adjustment of the quantity of insulin to be dispensed in a setting mode under the condition in which the upper limit of the quantity of insulin to be dispensed is preferentially selected as a setting value in the adjustment.

In order to generate a command for the execution of the preliminary step S200, a separate doctor mode selection switch may be provided to the key button unit 61 included in the circuit shown in Fig. 14. Alternatively, although the control circuit including the key button unit 61 and control unit 70 is still used, taking into consideration the security of the automatic syringe device, the above purpose may be accomplished without any design change. For instance, the key button unit 61 may be configured in such a fashion that a doctor mode selection command is generated when optional ones of the switches 61-1, 61-2, 61-3, and 61-4 are simultaneously depressed.

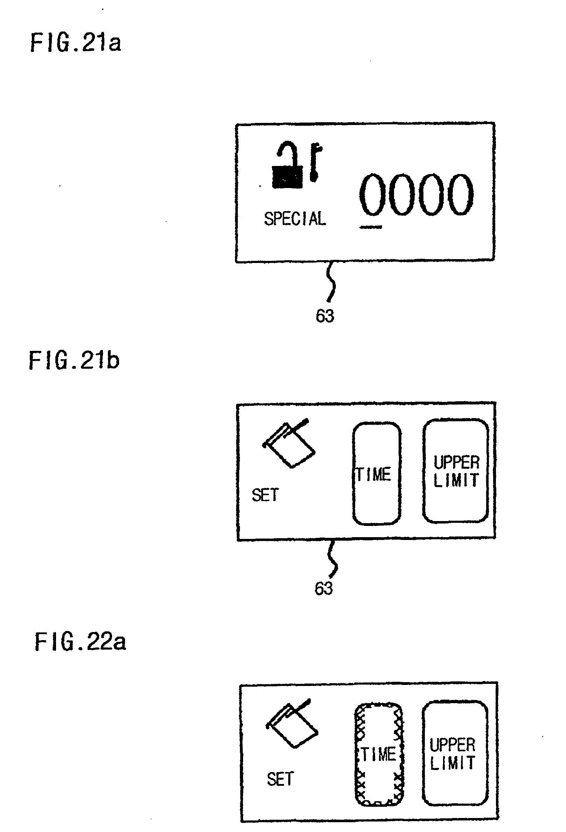

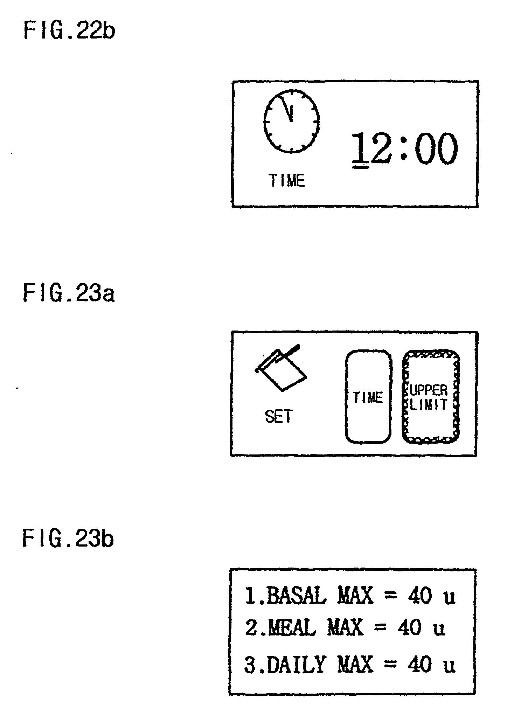

In accordance with the present invention, the ROM 65 is stored with an algorithm programmed in such a fashion that the control unit 70 recognizes an upper limit of the quantity of insulin, to be dispensed to a patient, set by an authorized doctor in the doctor mode while disregarding a quantity of insulin set beyond the set upper limit in the setting mode and preferentially selecting the set upper limit as a setting value. Preferably, a secret number is assigned in order to allow only the authorized doctor to execute the preliminary step S200 while preventing an optional execution of the preliminary step S200 by unauthorized doctors or persons. Now, a preferred embodiment of the present invention will be described in conjunction with Figs. 20 to 23b. When a doctor mode selection command is generated, a window informing of an execution of the preliminary step and requiring inputting of a secret number is displayed on the display unit 63, as shown in Fig. 21a. A cursor is positioned at a secret number window tab in the window so as to allow the user to input a secret number. When a secret number input is correct, a subsequent window for requiring a setting of a desired time and a desired upper limit of the insulin quantity to be dispensed is displayed on the display unit 63, as shown in Fig. 21b. In order to set a desired time, a "TIME" window tab is first selected from the window of Fig. 21b after positioning a cursor at the "TIME" window tab, as shown in Fig. 22a, thereby displaying a window of Fig. 22b. Using the "UP and "DOWN" switches 61-3 and 61-4 shown in Fig. 15, a desired time is set in the window of Fig. 22b. When the user depresses the select switch 61-2 after setting a desired time, an execution of first step S101 is begun. In order to set a desired upper limit of the insulin quantity to be dispensed, an "UPPER LIMIT" window tab is first selected from the window of Fig. 21b after positioning a cursor at the "UPPER LIMIT" window tab, as shown in Fig. 23a, and then depressing the select switch 61-2. Accordingly, a window is displayed, which includes a "BASAL MAX" window tab indicative of a maximum basal quantity of insulin to be dispensed, a "MEAL MAX" window tab indicative of a maximum quantity of insulin to be dispensed at the set mealtime, and a "DAILY MAX" window tab indicative of a maximum daily quantity of insulin to be dispensed, as shown in Fig. 23b. After selecting one of the window tabs shown in Fig. 23b, a desired upper limit associated with the selected window tab is set using the "UP and "DOWN" switches 61-3 and 61-4. In order to set the remaining upper limits, the remaining window tabs of Fig. 23b are selected one by one using the "NEXT" switch 61-1, and the same procedure as mentioned above is then executed. After completing a setting of desired upper limits, the user depresses the select switch 61-2. Thus, the preliminary step S200 is completed. When the select switch 61-2 is depressed, a command informing of the beginning of the mode selection step S101 is generated. The procedures following the preliminary step S200 are the same as those in Fig. 17. Accordingly, no further description will be made. Of course, when a value, which is beyond the upper limit set by the authorized doctor in the doctor mode, is set at step S102-4, the set upper limit is preferentially selected as a setting value in accordance with the present invention. Therefore, it is possible to prevent a low blood sugar level symptom caused by setting of an excessive quantity of insulin.

Although the preferred embodiments of the invention have been disclosed for illustrative purposes, those skilled in the art will appreciate that various modifications, additions and substitutions are possible, without departing from the invention as disclosed in the accompanying claims.

As apparent from the above description, the present invention provides a method for controlling the operation of a portable automatic insulin syringe device, which is capable of allowing only an authorized doctor to control the syringe device in a doctor mode in such a fashion that he sets an upper limit of the quantity of insulin to be dispensed to a patient, who uses the syringe device, while allowing the patient or other unauthorized person to adjust the quantity of insulin to be dispensed in a range not beyond the set upper limit of the insulin quantity. The selection of the doctor mode may be recognized in response to a simultaneous selection of optional ones among the switches conventionally equipped in the syringe device. Since the upper limit of the insulin quantity set in the doctor mode is preferential to a value set in a setting mode in accordance with the present invention, it is possible to eliminate harmful effects resulting from an excessive injection of insulin when insulin pumps are used.

## Claims

1. An automatic insulin syringe device having a control circuit (70) for administering pre-selected amounts of insulin to a patient, and
having means for selecting (61, 63) one of a plurality of operation modes (S101, S102) of the automatic insulin syringe device and means for executing (67, 68, 70) the selected operation mode,
**characterised by**
a doctor mode (S200) accessible by an authorized doctor, by using a secret code enabling access to said doctor mode and allowing the authorized doctor to set an upper limit of the quantity of insulin to be dispensed to the patient using said syringe device; and
means for determining (65) whether or not an operation mode of said syringe device other than said doctor mode is selected after said upper limit has been set; and
said means for executing said operation mode selected limiting an adjustment of the quantity of insulin to be dispensed to said upper limit.

## Patentansprüche

1. Automatische Insulininjektionsspritze, welche eine Steuerungsschaltung (70) zum Verabreichen vorbestimmter Insulinmengen an einen Patienten, und
eine Einrichtung (61, 63) zum Auswählen eines Betriebsmodus aus einer Mehrzahl von Betriebsmodi (S101, S102) der automatischen Insulininjektionsspritze, sowie eine Einrichtung (67, 68, 70) zum Ausführen des ausgewählten Betriebsmodus aufweist,
**gekennzeichnet durch**
einen Arztmodus (S200), der für einen autorisierten Arzt unter Verwendung eines geheimen Codes zugänglich ist und es dem autorisierten Arzt erlaubt, ein oberes Limit der Insulinmenge einzustellen, die dem Patienten bei Verwendung der Injektionsspritze verabreicht wird; und **durch**
eine Einrichtung (65) zum Bestimmen, ob ein anderer Betriebsmodus der Injektionsspritze als der Arztmodus ausgewählt ist, nachdem das obere Limit eingestellt wurde; sowie **dadurch**, daß
die Vorrichtung zum Ausführen des ausgewählten Betriebsmodus eine Einstellung der abzugebenden Insulinmenge auf das obere Limit begrenzt.

## Revendications

1. Seringue d'injection automatique d'insuline ayant un circuit de commande (70) pour administrer à un patient des quantités prédéterminées d'insuline, et ayant des moyens (61,63) pour sélectionner un d'une pluralité de modes opératoires de la seringue d'injection automatique d'insuline et des moyens (67,68,70) pour exécuter le mode opératoire sélectionné, **caractérisée par** :
- un mode médecin (S200) accessible par un médecin autorisé en utilisant un code secret donnant accès audit mode médecin et permettant au médecin autorisé de dispenser une limite supérieure de la quantité d'insuline à administrer au patient utilisant ladite seringue; et
- des moyens (65) pour déterminer si oui ou non un mode opératoire de ladite seringue autre que ledit mode médecin est sélectionné une fois que ladite limite supérieure a été administrée ; et lesdits moyens pour exécuter ledit mode opératoire sélectionné limitant un réglage de la quantité d'insuline à administrer à ladite limite supérieure.
